# EUROPEAN PATENT SPECIFICATION

(11) **EP 0 574 994 B1**
(45) Date of publication and mention of the grant of the patent: **12.03.1997**
(21) Application number: 93201677.7
(22) Date of filing: 11.06.1993
(51) Int. Cl.: C07C 5/27

(54) **Process for the conversion of a feedstock comprising linear olefins**
Verfahren zur Umsetzung einer geradkettigen Olefinen enthaltenden Beschickung
Procédé pour la conversion d'une change comprenant des oléfines linéaires

(30) Priority: 15.06.1992 EP 92201754
(43) Date of publication of application: 22.12.1993
(73) Proprietor: SHELL INTERNATIONALE RESEARCH MAATSCHAPPIJ B.V., 2596 HR Den Haag (NL)
(72) Inventor: Mooiweer, Hendrik Harm, NL-1031 CM Amsterdam (NL); Suurd, Jan, NL-1031 CM Amsterdam (NL); De Jong, Krijn Pieter, NL-1031 CM Amsterdam (NL)

(56) References cited:
- EP-A- 0 012 473
- EP-A- 0 501 077

## Description

The present invention relates to a process for the conversion of a feedstock comprising linear olefins into a product enriched in branched olefins.

It is foreseen that environmental legislation will bring about changes in gasoline manufacturing. It is expected that the concentration of lead anti-knock additives is to be reduced, which makes the required octane quality more difficult to attain. Components which are suitable for improving the octane quality of gasoline are certain branched ethers, e.g. methyl tertiary butyl ether (MTBE). It is known that such ethers can be formed by contacting branched olefins with methanol in the presence of a suitable acidic catalyst, such as sulphonic resins, phosphoric acid modified kieselguhr, silica/alumina and acid zeolites.

The object of the present invention is to obtain a product comprising branched olefins useful in the production of branched ethers, from a feedstock comprising linear olefins. Conversion of linear olefins generally involves a combination of operations including polymerization, dimerization and cracking. This means that in general not only the desired branched olefins are produced, but also a certain amount of, less desirable, heavier hydrocarbons.

In European patent specification 026 041 a process is described in which a mixed olefin feed is converted into an olefinic product enriched with C₄ and C₅ olefins comprising olefin isomers, which process comprises contacting the feed with an acid crystalline zeolite catalyst characterized by a pore opening of at least 5x10⁻¹⁰ m, a silica to alumina ratio of at least 12 and a constraint index within the range of 1 to 12 at an olefin pressure below 0.5 bar and a temperature between 204 °C and 316 °C. The process temperature would seem to be critical, and indeed above this temperature range there is a reduced selectivity for the olefin isomers using ZSM-5, the preferred catalyst of the described process. Moreover, at a temperature of 350 °C a large amount of relatively heavy hydrocarbons is produced.

It has now surprisingly been found that a feedstock comprising linear olefins can be converted into a product enriched in branched olefins at an olefin partial pressure of less than or equal to 0.5 bar without formation of large amounts of undesirable side-products, by contacting the feedstock with a tectometallosilicate having a certain specific crystal structure, i.e. a ferrierite crystal structure. Such process is particularly advantageous as it operates with greater selectivity to formation of branched olefins than with the known process.

Further, it has been found that the tectometallosilicate according to the present invention is relatively stable in the present process, i.e. its catalytic activity does not substantially change during a relatively long time.

In copending European patent application No. 0 501 577 a process is disclosed for converting a feedstock comprising linear olefins into a product enriched in branched olefins at an olefin partial pressure of more than 0.5 bar.

The present invention relates to a process for the conversion of a feedstock comprising linear olefins into a product enriched in branched olefins, which process comprises contacting the feedstock with a tectometallosilicate having a ferrierite crystal structure, at a temperature between 150 and 450 °C, an olefin partial pressure of less than or equal to 0.5 bar and a maximum total pressure of 25 bar.

The tectometallosilicate having a ferrierite structure to be employed in the process of the present invention, has in its substantially template free, hydrogen form an X-ray diffraction pattern which contains at least the d-spacings (in Angstrom) as set forth below in table 1.

The d-spacing in Angstrom is the value of the interplanar spacing of the crystal and is calculated from the measured theta (Bragg angle) by using the Bragg equation. The relative intensity of the diffraction peak corresponding to each d-spacing is given as a value: w (weak), m (medium), s (strong) or vs (very strong), determined relative to the intensity of the main diffraction peak.

**Table 1:**

| X-ray diffraction pattern of ferrierite | |
|---|---|
| d | Relative intensity |
| 11.3 ± 0.3 | w-m |
| 9.5 ± 0.1 | s-vs |
| 7.08 ± 0.04 | m |
| 6.96 ± 0.04 | m |
| 3.98 ± 0.02 | m-s |
| 3.76 ± 0.02 | m |
| 3.54 ± 0.02 | m-s |
| 3.47 ± 0.02 | m-s |
| 3.13 ± 0.02 | w-m |
| 3.05 ± 0.02 | w-m |

Examples of tectometallosilicates having a ferrierite structure are ferrierite, FU-9, ISI-6, Nu-23, ZSM-21, ZSM-35 and ZSM-38. The tectometallosilicates which can be employed, contain silicon and one or more from the group consisting of iron, gallium and aluminium. In general, the tectometallosilicate will mainly contain silicon and aluminium. Preferably, the tectometallosilicate is in the hydrogen form. Tectometallosilicates which have shown an especially high stability, are tectometallosilicates having a relatively high silicon to aluminium molar ratio, e.g. a silicon to aluminium atomic ratio of more than 10, preferably more than 15. The tectometallosilicate may have a silicon to aluminium atomic ratio of more than 30. A process for preparing tectometallosilicate which can be suitably applied in the process of the present invention is described in European patent specification No. 012 473. The tectometallosilicate can be used as such. However, often it is economically more attractive to use it in combination with a binder. Binders which can suitably be applied are amorphous refractory oxides such as alumina, silica or silica/alumina.

The tectometallosilicate may be surface moderated, by deactivation or removal of some or all surface acid sites at the external surface by techniques known in the art. Suitable techniques include those disclosed in S. Bhatia, "Zeolite Catalysis: Principles and Applications", CRC Press, Boca Raton (Florida), 1990. For example the catalyst may be treated with ethylenediamine tetraacetic acid (EDTA) as disclosed in D.W. Breck, "Zeolite Molecular Sieves - Structure, Chemistry and Use" John Wiley & Sons, New York 1974. The catalyst may optionally be used in the form of crystals having a low ratio of crystal surface area to pore surface area.

The feedstock which is to be used in the present invention comprises linear olefins, suitably linear olefins containing between 4 and 10 carbon atoms. Higher olefins, e.g. olefins comprising up to 20 carbon atoms, and/or lower olefins, e.g. ethene and/or propene, can be present in the feedstock. Higher olefins could for example be present if part of the product obtained is recycled to the conversion step. Suitable feedstocks for the process of the present invention are hydrocarbon streams as obtained in catalytic cracking or steam cracking. Suitably, the feedstock can comprise linear olefins containing between 4 and 8 carbon atoms. Preferred feedstocks comprise linear olefins containing 4 or 5 carbon atoms. The feedstock can further contain a certain amount of paraffins. Paraffins commonly present include butane and pentane.

Feedstocks containing excess paraffins may conveniently be passed over an olefins/paraffins separating unit prior to conversion.

Surprisingly it has also been found that in a conversion process using the tectometallosilicate of the present invention it is possible to recycle to the conversion step substantially all the unconverted linear olefin fraction of the product due to the low level of butane by product in the conversion step. Processes using for example ZSM-5 as catalyst should include a bleed stream in a recycle to the conversion step for control of butane level. In such processes the fraction of the recycle bled is sufficiently high that unconverted olefins in the bleed may not economically be isolated and returned to the conversion unit.

In a conversion process using the tectometallosilicate of the present invention a bleed stream in the recycle to conversion step may be passed for example to a butane/butene extractive distillation unit and the olefin fraction returned to the conversion unit. Alternatively the distillation unit may be omitted, nevertheless incurring low wastage of linear olefins since the fraction of the recycle bled is significantly less than with processes using ZSM-5.

It has moreover been found that a conversion process using the tectometallosilicate under the conditions of the present invention yields still less butane by-product than had previously been found using the conditions of the copending European patent application No. 0 501 577.

The product obtained in the process of the present invention is enriched in branched olefins. From a commercial point of view, it is advantageous to produce as much branched olefins as possible. Suitably, the product obtained comprises at least 20 % by weight of branched olefins, based on the amount of olefins present in the feedstock; preferably, the product obtained comprises at least 30 % by weight of branched olefins. Further, it is preferred in a process according to the present invention, to produce only a small amount of heavier hydrocarbons such as aromatics. Preferably, a product is obtained comprising less than 10 % by weight of aromatics based on the total amount of liquid product, i.e. product which is in the liquid state under normal conditions; most preferably, the product comprises less than 5 % by weight of aromatics.

The product obtained can be suitably used for the production of highly branched ethers. Before the product is used in such ether producing process, it is preferred to separate off a heavy fraction from the product obtained, and to recycle at least part of this heavy fraction to the conversion step. The heavy fraction suitably comprises hydrocarbons containing at least 5 carbon atoms. The product remaining after the heavy fraction is separated off, is contacted with a lower alcohol in the presence of a suitable acidic catalyst. In this process step mainly branched olefins react. The branched ethers produced are removed, and the remaining hydrocarbon mixture rich in linear olefins can be recycled to the conversion step.

Process conditions which can be suitably applied, comprise a temperature between 150 and 450 °C, an olefin partial pressure of less than or equal to 0.5 bar and a maximum total pressure of 25 bar. Preferred process conditions comprise a temperature between 290 and 450 °C, more preferably between 300 and 450 °C, for example between 320 and 430 °C and an olefin partial pressure of between 0.01 and 0.50 bar, most preferably between 0.1 and 0.5 bar.

The invention will now be further elucidated with the aid of the following examples.

### Example 1

Commercially available ferrierite ex Toyo Soda, which ferrierite was in the ammonium form and had a silicon to aluminium atomic ratio of 9, was pressed, crushed and sieved to obtain a 30-80 mesh size fraction. The particles thus obtained were calcined for 2 hours at 540 °C.

### Example 2

544 g silica gel was combined with a solution of 54 g sodium hydroxide in 1000 g water and the resulting mixture was homogenized. A second solution comprising 61.4 g aluminium sulphate (Al₂(SO₄)₃.18 H₂O), 256 g sodium sulphate and 1000 g water was added under stirring. Finally, 218 g pyridine dissolved in 1152 g water were admixed giving a reaction gel of the composition (on a molar basis) 93.5 SiO₂ . 1 Al₂O₃ . 7.4 Na₂O . 19.6 Na₂SO₄ . - 30 pyridine . 1938 H₂O. This reaction gel was kept at 150 °C for a period of 75 hours until a crystalline compound was obtained. After synthesis the crystalline compound produced was separated from the reaction mixture by filtration, water washed and dried at 120 °C.

The dried compound was calcined at 500 °C, cooled down and ion exchanged such that the compound is brought in the ammonium form. The solid product was separated from the liquid by filtration, water washed, dried at 120 °C and subsequently calcined at 500 °C.

The product of the synthesis was determined by X-ray diffraction to be essentially ferrierite. The silicon to aluminium atomic ratio was found to be 36.

Before employment in the process of the present invention the ferrierite powder was pressed, crushed and sieved in order to obtain a 30-80 mesh fraction.

### Example 3

Commercially available ZSM-5, CBV 3020 ex Conteka, was pressed, crushed and sieved in order to obtain particles having a 30-80 mesh size fraction. The particles thus obtained were calcined for 2 hours at 540 °C.

### Example 4

A feedstock as described in Table 2, is contacted with ferrierite and ZSM-5, prepared as described in Examples 1 and 3, respectively. The process is carried out at a temperature of 350 °C, an olefin partial pressure of 0.3 and 0.4 bar respectively, a weight hourly space velocity of 2 kg/kg/hour and a total pressure of 1.1 to 1.4 bar. The products obtained after 50 hours on-stream are described in Table 3, in % by weight of product obtained.

**Table 2:**

| Feedstock properties | |
|---|---|
| n-butene (% by weight) | 99.42 |
| iso-butene (% by weight) | 0.44 |
| butane (% by weight) | 0.14 |

**Table 3:**

| Product obtained | | |
|---|---|---|
| | Catalyst | |
| | Ferrierite (olefin partial pressure 0.3 bar) | ZSM-5 (olefin partial pressure 0.4 bar) |
| hydrogen | - | 0.06 |
| C₁-C₂ paraffins | 0.00 | 0.11 |
| ethene | 0.00 | 0.58 |
| propane | 0.00 | 9.58 |
| propene | 0.61 | 1.38 |
| butane | 0.82 | 26.31 |
| n-butene | 56.32 | 0.85 |
| iso-butene | 40.85 | 0.85 |
| C₅⁺ | 1.4 | 60.28 |

### Example 5

Ferrierite samples prepared as described in Example 2, are contacted at a temperature of 350 °C, an olefin partial pressure of 0.3 bar and 1.1 bar respectively, a weight hourly space velocity of 2 kg/kg/hour and a total pressure of 1.1 to 1.4 bar with the feedstock as described in Table 2. The products obtained after 480 hours on-stream are described in Table 4.

**Table 4:**

| Product obtained | | |
|---|---|---|
| | olefin partial pressure | |
| | 0.3 bar | 1.1 bar |
| C₁-C₂ paraffins | 0.00 | 0.00 |
| ethene | 0.00 | 0.02 |
| propane | 0.00 | 0.03 |
| propene | 0.48 | 0.94 |
| butane | 0.47 | 1.31 |
| n-butene | 50.30 | 41.37 |
| iso-butene | 47.95 | 40.43 |
| C₅⁺ | 0.8 | 15.9 |

### Example 6

Ferrierite prepared as described in Example 1, is contacted at temperatures of 350 °C and 300 °C, an olefin partial pressure of 0.3 bar, a weight hourly space velocity of 2 kg/kg/hour and a total pressure of 1.1 to 1.4 bar with the feedstock as described in Table 2. The products obtained after 50 hours on stream are described in Table 5.

**Table 5:**

| Product obtained | | |
|---|---|---|
| | Temperature | |
| | 350 °C | 300 °C |
| C₁-C₂ paraffins | 0.00 | 0.00 |
| ethene | 0.00 | 0.00 |
| propane | 0.00 | 0.00 |
| propene | 0.61 | 0.98 |
| butane | 0.82 | 1.40 |
| n-butene | 56.32 | 64.63 |
| iso-butene | 40.85 | 30.89 |
| C₅⁺ | 1.4 | 2.1 |

## Claims

1. Process for the conversion of a feedstock comprising linear olefins into a product enriched in branched olefins, which process comprises contacting the feedstock with a tectometallosilicate having a ferrierite crystal structure, at a temperature between 150 and 450 °C, an olefin partial pressure of less than or equal to 0.5 bar and a maximum total pressure of 25 bar.

2. Process according to claim 1, wherein the feedstock comprises linear olefins containing between 4 and 10 carbon atoms.

3. Process according to claim 1 and/or 2, wherein the process is carried out at a temperature between 290 and 450 °C and an olefin partial pressure of between 0.01 and 0.5 bar.

4. Process according to any one of the preceding claims, wherein the product comprises at least 30 % by weight of branched olefins, based on the amount of olefins present in the feedstock.

5. Process according to any one of the preceding claims, wherein the product comprises less than 10 % by weight of aromatics, based on the total amount of liquid product.

6. Process according to any one of the preceding claims, wherein the tectometallosilicate has a silicon to aluminium atomic ratio of more than 15.

7. Process according to claim 6 wherein the tectometallosilicate has a silicon to aluminium atomic ratio of more than 30.

8. Process according to any one of the preceding claims, wherein the tectometallosilicate is in the hydrogen form.

9. Process according to any one of the preceding claims, wherein the tectometallosilicate is surface-moderated.

10. Process according to any one of the preceding claims, wherein a heavy fraction is separated off from the product obtained, at least part of which heavy fraction is optionally recycled to the conversion step.

11. Process according to claim 10, wherein the heavy fraction comprises hydrocarbons containing at least 5 carbon atoms.

12. Process according to any one of the preceding claims, wherein substantially all of the unconverted linear olefin fraction of the product is separated off and recycled to the conversion step.

## Patentansprüche

1. Verfahren zur Umwandlung eines lineare Olefine umfassenden Einsatzmaterials in ein an verzweigten Olefinen angereichertes Produkt, welches Verfahren ein In-Kontakt-Bringen des Einsatzmaterials mit einem Tectometallsilicat mit einer Ferrierit-Kristallstruktur bei einer Temperatur zwischen 150 und 450°C, einem Olefinpartialdruck von kleiner als oder gleich 0,5 bar und einem maximalen Gesamtdruck von 25 bar umfaßt.

2. Verfahren nach Anspruch 1, worin das Einsatzmaterial lineare Olefine mit zwischen 4 und 10 Kohlenstoffatomen umfaßt.

3. Verfahren nach Anspruch 1 und/oder 2, worin das Verfahren bei einer Temperatur zwischen 290 und 450°C und einem Olefinpartialdruck zwischen 0,01 und 0,5 bar ausgeführt wird.

4. Verfahren nach einem der vorstehenden Ansprüche, worin das Produkt wenigstens 30 Gew.-% an verzweigten Olefinen umfaßt, bezogen auf die im Einsatzmaterial vorliegende Olefinmenge.

5. Verfahren nach einem der vorstehenden Ansprüche, worin das Produkt weniger als 10 Gew.-% an Aromaten umfaßt, bezogen auf die Gesamtmenge des flüssigen Produktes.

6. Verfahren nach einem der vorstehenden Ansprüche, worin das Tectometallsilicat ein Silicium/Aluminium-Atomverhältnis von über 15 aufweist.

7. Verfahren nach Anspruch 6, worin das Tectometallsilicat ein Silicium/Aluminium-Atomverhältnis von über 30 aufweist.

8. Verfahren nach einem der vorstehenden Ansprüche, worin das Tectometallsilicat in der Wasserstoffform vorliegt.

9. Verfahren nach einem der vorstehenden Ansprüche, worin das Tectometallsilicat oberflächenmoderiert ist.

10. Verfahren nach einem der vorstehenden Ansprüche, worin eine schwere Fraktion von dem erhaltenen Produkt abgetrennt wird, wobei wenigstens ein Teil der schweren Fraktion gegebenenfalls zur Umwandlungsstufe zurückgeführt wird.

11. Verfahren nach Anspruch 10, worin die schwere Fraktion Kohlenwasserstoffe mit wenigstens 5 Kohlenstoffatomen enthält.

12. Verfahren nach einem der vorstehenden Ansprüche, worin im wesentlichen die Gesamtmenge der nicht umgewandelten linearen Olefinfraktion des Produktes abgetrennt und in die Umwandlungsstufe zurückgeführt wird.

## Revendications

1. Procédé de conversion d'une charge de départ comprenant des oléfines linéaires en un produit enrichi en oléfines ramifiées, caractérisé en ce que l'on met la charge de départ en contact avec un tectométallosilicate possédant une structure à cristal de ferriérite, à une température comprise entre 150 et 450°C, sous une pression partielle d'oléfine inférieure ou égale à 0,5 bar et une pression totale maximale de 25 bars.

2. Procédé suivant la revendication 1, caractérisé en ce que la charge de départ comprend des oléfines linéaires contenant de 4 à 10 atomes de carbone.

3. Procédé suivant les revendications 1 et/ou 2, caractérisé en ce qu'on l'entreprend à une température comprise entre 290 et 450°C et sous une pression partielle d'oléfine comprise entre 0,01 et 0,5 bar.

4. Procédé suivant l'une quelconque des revendications précédentes, caractérisé en ce que le produit comprend au moins 30% en poids d'oléfines ramifiées, sur base de la quantité d'oléfines présentes dans la charge de départ.

5. Procédé suivant l'une quelconque des revendications précédentes, caractérisé en ce que le produit comprend moins de 10% en poids de composés aromatiques, sur base de la quantité totale de produit liquide.

6. Procédé suivant l'une quelconque des revendications précédentes, caractérisé en ce que le tectométallosilicate possède un rapport atomique silicium à aluminium supérieur à 15.

7. Procédé suivant la revendication 6, caractérisé en ce que le tectométallosilicate possède un rapport atomique silicium à aluminium supérieur à 30.

8. Procédé suivant l'une quelconque des revendications précédentes, caractérisé en ce que le tectométallosilicate se présente sous la forme hydrogène.

9. Procédé suivant l'une quelconque des revendications précédentes, caractérisé en ce que le tectométallosilicate est à surface modérée.

10. Procédé suivant l'une quelconque des revendications précédentes, caractérisé en ce qu'une fraction lourde est séparée du produit obtenu, au moins une partie de cette fraction lourde étant éventuellement recyclée à l'étape de conversion.

11. Procédé suivant la revendication 10, caractérisé en ce que la fraction lourde comprend des hydrocarbures contenant au moins 5 atomes de carbone.

12. Procédé suivant l'une quelconque des revendications précédentes, caractérisé en ce que sensiblement la totalité de la fraction des oléfines linéaires non converties du produit est séparée et recyclée à l'étape de conversion.
